# EUROPEAN PATENT APPLICATION

(11) **EP 4 541 724 A1**
(43) Date of publication of application: **23.04.2025**
(21) Application number: 23205004.7
(22) Date of filing: 20.10.2023
(51) Int. Cl.: B65D 1/02, B65D 1/40, A61J 1/00, B29C 45/44, B29L 31/00

(54) **CONTAINER FOR PHARMACEUTICAL, MEDICAL, COSMETIC, DIETETIC AND FOOD PRODUCTS AND METHOD AND APPARATUS FOR PRODUCING SUCH CONTAINER**

(71) Applicant: Gerresheimer Vaerloese A/S, 3500 Værløse (DK)
(72) Inventor: Düring Darzanos, Hans Christian, 2450 København SV (DK); Dirk, Wolfgang, 22359 Hamburg (DE); Kjaerlund, Jan, 3650 Oestykke (DK)
(74) Representative: Schmid, Nils T.F.

(57) **Abstract**

The invention relates to a container, in particular a primary packaging container, for pharmaceutical, medical, cosmetic, dietetic and/or food products, comprising an interior surface with at least one undercut section, wherein at least a portion of the container has an average wall thickness of maximally 0,8 mm, preferably of between 0,2 mm and 0,8 mm, more preferably between 0,3 mm and 0,7 mm, most preferably between 0,4 mm and 0,6 mm.

## Description

The present invention relates to a container for pharmaceutical, medical, cosmetic, dietetic and/or food products, and to a method and apparatus for injection molding of a container for pharmaceutical, medical, cosmetic, dietetic and/or food products. In particular, the invention relates to respective containers, in particular primary packaging containers, for pharmaceutical and medical products. More in particular, the invention relates to tablet, eye-drop and nasal spray containers, such as vials, cartridges, ampoules, bottles, or syringes.

Today, with a certain range of volumetric capacity and undercut ratio (see definition below), such containers are produced by injection blow molding, extrusion blow molding, or compression blow-forming. In particular, the range of volume capacity of containers being produced with such methods is between 10 and 2.000 milliliters, in particular between 20 and 1.000 milliliters, more in particular between 30 and 500 milliliters, even more in particular between 40 and 250 milliliters, most in particular between 40 and 150 milliliters, for example between 40 and 80 milliliters, such as 60 milliliters. The range of undercut ratio of containers being produced with such methods is in particular between 1.05 to 2, in particular between 1.10 to 1.5, more in particular between 1.15 to 1.25, for example about 1.2.

An example for injection blow molding of such containers is described in EP 1 896 236 B1. Therein, a pre-form is created by injecting material into a molding chamber formed between a preform cavity member defining the exterior surface of the pre-form and a pre-form core member defining the interior surface of the preform. In a second step, the pre-form core member is moved with the pre-form thereon into a final-form cavity member defining the outer surface of the final container, where hot gas is fed through the pre-form core to blow the preform towards the final-form cavity member. In a third step, the pre-form core is removed from the interior of the container and the final-form cavity opens so that the final container can be ejected. This three step procedure allows to produce containers for pharmaceutical and medical products within the above mentioned range of volumetric capacity and undercut ratio.

Disadvantages of this method are however long cycle times increasing costs and energy consumption as well as heterogenic and larger wall thickness (see for example figures 8a to 8c of EP 1 896 236 B1), which increases costs and material consumption.

Recent approaches of optimizing the mold design and process control allowed to reduce the material consumption by 25 %. Further, improving the machine management allowed to reduce the energy consumption for melting the material by 35 to 50 %. Nevertheless, there is a need to further reduce energy and material consumption to enhance the sustainability of such containers.

It is an object of the present invention to overcome the disadvantages from the prior art, in particular to provide a container for pharmaceutical, medical, cosmetic, dietetic and/or food products, as well as a method and an apparatus for injection molding such container with enhanced sustainability.

This object is solved by the features of the independent claims.

The present invention comprises six independent claims relating to six aspects of the invention all having in common that they relate to containers, in particular to primary packaging containers, for pharmaceutical, medical, cosmetic, dietetic and/or food products or to a method or apparatus for injection molding of such containers. Within the meaning of the present invention, medical products shall preferably be understood to also comprise healthcare products and diagnostic products. Further, within the meaning of the present invention, dietetic products shall be understood to also encompass vitamins, mineral supplements and herbals, such as cannabis. Specific requirements for such containers are high purity, protection of products to be stored therein from change, low permeability, low adhesion, and flat interieur surfaces. In particular, a packaging product for pharmaceutical, medical, cosmetic, dietetic and/or food products shall be understood to be a container meeting the requirements of the European Union and/or of the United States of Amerika for such products at the filing date of the present application. For example, a container within the meaning of the invention can be a container having a moisture vapor transmission rate as being defined as requirement for pharmaceutical use of immediately primary packed solid dosage formulations, in particular as requirement in US 671). The investors have found that an advantageous moisture vapor transmission rate can be achieved by producing the container, and preferably its closure, from Polypropylene, in particular in that the container consists to at least 50%; 60%, 70%, 80, 90%, 95%, 99% or 100 % by weight of Polypropylene. In particular, a container being suitable for storing pharmaceutical, medical, cosmetic, dietetic and/or food products is a container being made of a pharmaceutical, medical, cosmetic, dietetic and/or food acceptable material or coated with such material, i.e. material not harming these products, in particular not harming these products in that they can become dangerous when applied to humans or animals.

Particularly high requirements are defined with respect to the purity of such containers in that they must be substantially free of particle and endotoxin loads. In particular in cases of containers made of polymers, electrostatic charges caused by the process may attract particles contaminating the container and thus making it unsuitable for pharmaceutical and medical products. To avoid or reduce particle and endotoxin loads, it is known to wash or apply temperatures of up to 300° to the containers. However, washing leads, in addition to energy consumption, to water consumption rendering the process less sustainable. Applying temperatures of up to 300° in turn would melt polymer containers, such as polyethylene containers, and is thus recently only applied to glass containers. However, glass containers have several disadvantages, such as being susceptible the breakage (thus requiring special and expensive processing and handling equipment over its entire lifetime from manufacturing of the container over its filling with products to its transportation und use) and having high tolerances (leading to high material and energy consumption). Other methods of producing containers for medical and pharmaceutical products achieve the high purity requirements by producing and handling containers in a clean room of class 100 or blowing inert gas around them during manufacturing as explained for example in DE 102 54 762 A1.

Protection of products to be stored in the container from change, in particular by external influence and/or impact, is important to avoid any risks when consuming products stored in the container.

Low permeability is of particular importance to avoid contamination or leakage of the products stored in the container.

Low adhesion, and flat interieur surfaces is of particular importance to reduce the risk that impurities, such as particle and endotoxin loads adhere in the container and/or remain therein even after purification.

The above described requirements are particularly high for containers for pharmaceutical and medical products and are defined for instance in pharmaceutical books. For particular products to be stored in the container, these requirements can be particularly high. For primary packaging containers, i.e. containers coming in direct contact with the product, these requirements are even higher as primary packaging containers come in direct contact with the product. The highest requirements particularly apply to primary packaging containers for pharmaceutical or medical products.

A container for pharmaceutical, medical, cosmetic, dietetic and/or food products within the meaning of the present invention is in particular to be understood as a container meeting at least one, preferably multiple, most preferably all, of the above defined requirements for such containers. Preferably, such container is a vial, cartridge, ampoule, bottle or syringe, more preferably a vial, cartridge or ampoule. In particular, a container within the meaning of the present invention has a certain range of volumetric capacity and/or undercut ratio (see definition below). In particular, the range of volume capacity of containers being produced with such methods is between 10 and 2.000 milliliters, in particular between 20 and 1.000 milliliters, more in particular between 30 and 500 milliliters, even more in particular between 40 and 250 milliliters, most in particular between 40 and 150 milliliters, for example between 40 milliliters and 80 milliliters, such as 60 milliliters. Containers for pharmaceutical, medical, cosmetic, dietetic and/or food products within the meaning of the present invention often require relatively large undercut ratios within the interieur surface of the container so that, if they are produced by a two-step injection blowing method, they cannot be ejected by deforming the container (forced ejection). Therefore, in the prior art, such containers are produced by three-step injection molding, such as injection blow molding. The range of undercut ratio in containers according to the present invention is preferably between 1.05 to 2, more in particular between 1.10 to 1.5, most in particular between 1.15 to 1.25, for example about 1.2.

The containers according to the present invention comprise an interieur surface with at least one undercut section. An undercut section in the interieur surface of the container is in particular to be understood as a section of the container changing, in particular reducing, its cross section in that a solid core member, in particular a core member which cannot change its exterior dimension, defining the interior cannot be removed from the interieur. Preferably, a container within the meaning of the present invention extends along a longitudinal axis and comprises a closed bottom at one end of a longitudinal axis and an open top at the opposite end of the longitudinal axis. The direction from the closed bottom to the open top can be referred to as opening direction. For such container, an undercut section can in particular be understood as a section of the interior surface changing its cross section in opening direction in that a solid core member, in particular a core member which cannot change its exterior dimension, defining the interior cannot be removed in opening direction. This can be the case if the shape of the cross section changes, for example from a circular shape to an oval or rectangular shape, and/or if the cross section is reduced in opening direction. However, preferably, an undercut section within the meaning of the present invention is realized by a reduction of cross section in opening direction. Preferably, the interior of the container is at least partially rotationally shaped in the region of the undercut section, wherein the diameter of the interieur surfaces is reduced in opening direction. The reduction of diameter can be continuous, for example by means of a truncated cone shape of the undercut section, in particular of the complete interieur surface, tapering in opening direction, and/or by an abrupt diameter step, for instance by means of a body portion extending from the closed bottom towards the open top and a neck portion extending between the body portion and the open top, wherein the neck portion has a smaller diameter than the body portion, wherein the body portion can be connected with the neck portion by a shoulder portion with the shape of a perforated disc. Preferably, the interior of a container according to the present invention comprises at least a cylindrical body portion extending from the closed bottom towards the open top and a cylindrical neck portion extending between the body portion and the open top, wherein the diameter of the neck portion is smaller than the diameter of the body portion thereby exhibiting an undercut section between the body portion and the neck portion. The undercut section can be realized for example by a shoulder portion with perforated disc shape or with truncated cone shape tapering towards the open top. As described in detail below, a safety-lock portion, preferably having at the interieur surface a cylindrical shape, can be located between the body portion and the neck portion, wherein the undercut section can extend between the safety-lock portion and the body portion, between the safety-lock portion and the neck portion or can be divided into two undercut sections, namely one lower undercut section extending between the body portion and the safety-lock portion and an upper undercut section extending between the safety-lock portion and the neck portion. Additionally or alternatively, an undercut section can be located between the neck portion and a mouth portion, being preferably cylindrically shaped at the interior surface and extending between the neck portion and the open top, wherein the mouth portion has a smaller diameter than the neck portion.

The previously described volume capacity of the container can in particular be defined by the volume defined by the interieur surface of the container between the closed bottom and the open top. The previously described undercut ratio can be calculated by measuring the smallest diameter of the interior surface and the largest diameter of the interior surface, which extends between the smallest diameter and the closed bottom, preferably wherein the largest diameter divided by the smallest diameter defines the undercut ratio. In the case of an interior surface extending between the closed bottom and the open top continuously with the shape of a truncated cone, the undercut ratio can be calculated by dividing the diameter of the interieur surface at the closed bottom by the diameter of the interieur surface at the open top. In the case of a container with a cylindrical body portion, a cylindrical safety-lock portion, a shoulder portion with a truncated cone shape tapering towards the open top and being divided by the safety-lock portion, a cylindrical neck portion and a cylindrical mouth portion, wherein the mouth portion has the smallest diameter and the body portion has the largest diameter, the undercut ratio can be calculated by dividing the diameter of the interieur surface of the body portion by the diameter of the interieur surface the mouth portion. Further, in such case, the undercut ratio between individual parts, for instance between the body portion and the neck portion, wherein the neck portion has a smaller diameter than the body portion, can be calculated by dividing the diameter of the interieur surface of the body portion by the diameter of the interieur surface of the neck portion.

According to one aspect of the invention a primary packaging container for pharmaceutical, medical, cosmetic, dietetic and/or food products, is provided, which comprises an interior surface with at least one undercut section, wherein at least a portion of the container has an average wall thickness of maximally 0,8 mm, preferably maximally 0,7 mm, more preferably maximally 0,6 mm. Preferably, the average wall thickness of the at least one portion is between 0,2 mm and 0,8 mm, more preferably between 0,3 mm and 0,7 mm, most preferably between 0,4 mm and 0,6 mm. Additionally or alternatively, the at least one portion is selected from a closed bottom, a body portion extending between the closed bottom and an open top, a neck portion extending between the body portion and the open top, a safety-lock portion extending between the body portion and the neck portion and a shoulder portion extending between the body portion and the safety-lock portion. More preferably, at least two, at least three, at least four or at least five of the previously mentioned portions have the previously described average wall thickness, particularly preferred of between 0,4 mm and 0,6 mm. In the following, some of the portions of the container will be described with respect to this aspect of the invention. However, it should be clear that all portions of the container according to this aspect of the invention can be realized as described above and below with respect to further aspects and embodiments of this invention and their embodiments, wherein the features attributed to the respective aspects and embodiments of the invention may or may not be realized.

According to a first embodiment, the interior of the container extends, is in particular at least partially rotationally shaped, along a longitudinal axis and comprises a closed bottom at one end of the longitudinal axis and an open top at the opposite end of the longitudinal axis. Preferably the closed bottom is circular shaped and particularly extends orthogonal to the longitudinal axis. The open top is preferably circular shaped, in particular is a circular opening, extending preferably orthogonal to the longitudinal axis and/or parallel to the closed bottom.

According to a second embodiment, which can be combined with the first embodiment, the at least one portion is a closed bottom, preferably the closed bottom of the first embodiment, of the container, preferably wherein the average wall thickness of the body portion is maximally 0,75 mm, more preferably maximally 0,65 mm, most preferably maximally 0,6 mm. Particularly preferred, the average wall thickness of the closed bottom is between 0,2 mm and 0,8 mm, more preferably between 0,35 mm and 0,7 mm, most preferably between 0,5 mm and 0,6 mm, for example 0,54 mm. The closed bottom is preferably circular shaped, preferably having a diameter between 20 mm and 90 mm, preferably between 30 mm and 60 mm, more preferably between 35 mm and 45 mm, even more preferably between 37.5 mm and 42.5 mm, for example 39,4.

According to a third embodiment, which can be combined with the first embodiment and/or second embodiment, the at least one portion is a body portion extending from a closed bottom of the container towards an open top of the container, preferably wherein the average wall thickness of the body portion is maximally 0,7 mm, more preferably maximally 0,6 mm, most preferably maximally 0,5 mm. Particularly preferred, the average wall thickness of the body portion is between 0,2 mm and 0,8 mm, more preferably between 0,3 mm and 0,6 mm, most preferably between 0,4 mm and 0,5 mm. Preferably, the body portion extends from the closed bottom towards the open top along between 20% and 90%, preferably between 30% and 80%, more preferably between 40 % and 70%, most preferably between 50% and 60 %, of the axial extension of the container. Preferably, the body portion comprises a cylindrical jacket extending from a circular bottom, preferably the circular bottom according to the first or second embodiment, towards the open top, in particular wherein the cylindrical jacket and the circular bottom together have the shape of a cup. The cylindrical jacket of the body portion preferably has the shape of an, in particular perfect, hollow cylinder with an inside diameter and an outside diameter being separated by each other by an, in particular constant, wall thickness. As described in detail above and below, the inside surface, and preferably also the outside surface, of the body portion is preferably free, of any recesses, protrusions and/or threads. As will be described below in detail, by use of a collapsible core for injection molding of the container, thin fins are generated in the interieur of the container. Thus, within the meaning of the present invention, an inside surface being described to be free of any recesses, protrusion and/or threads shall preferably not exclude such thin fins. Preferably, the cylindrical jacket of the body portion has an inside diameter between 20 mm and 90 mm, preferably between 30 mm and 60 mm, more preferably between 35 mm and 45 mm, even more preferably between 37.5 mm and 42.5 mm, for example 39,4 mm and/or extends along the longitudinal axis between 10 mm and 60 mm, preferably between 15 mm and 45 mm, more preferably between 20 mm and 35 mm, most preferably between 25 mm and 30 mm, for example along 27 mm. Preferably the above described average wall thickness of the body portion is calculated over the wall thickness along the entire extension of the body portion in circumferential direction and along the longitudinal axis. It shall be clear hat, where the present disclosure discloses wall thicknesses for different parts of the container, the average wall thickness is preferably meant which can be calculated as described for the body portion.

In a fourth embodiment, preferably according to any of the first to the third embodiment, the at least one portion is a neck portion extending between the body portion and the open top of the container, wherein the inside of the neck portion is preferably cylindrically shaped. Preferably, the average wall thickness of the neck portion is maximally 0,7 mm, more preferably maximally 0,6 mm, most preferably maximally 0,5 mm. Particularly preferred, the average wall thickness of the neck portion is between 0,2 mm and 0,8 mm, more preferably between 0,3 mm and 0,6 mm, most preferably between 0,4 mm and 0,5 mm. Preferably, the neck portion extends between the body portion and the open top along between 10% and 50%, preferably between 15% and 40%, more preferably between 20 % and 35%, most preferably between 25% and 30%, of the axial extension of the container. Particularly preferred, the neck portion comprises a cylindrical jacket in particular having the shape of an, in particular perfect, hollow cylinder with an inside diameter and an outside diameter being separated by each other by an, in particular constant, wall thickness. As described in detail above and below, the neck portion can comprise thread means on the outside surface of the cylindrical jacket, wherein the inside surface is preferably free of any recesses, protrusions and/or threads. Preferably, the cylindrical jacket of the neck portion has an inside diameter between 10 mm and 55 mm, preferably between 20 mm and 45 mm, more preferably between 25 mm and 40 mm, even more preferably between 30 mm and 35 mm, for example of 33 mm and/or extends along the longitudinal axis between 5 mm and 35 mm, preferably between 7,5 mm and 25 mm, more preferably between 10 mm and 20 mm, most preferably between 12,5 mm and 17,5 mm, for example along 14,4 mm.

According to a fifth embodiment, which can be combined with any of the first to the fourth embodiment, a closed bottom of the container has a different average wall thickness than a body portion extending from the closed bottom towards an open top of the container or a neck portion extending between the body portion and the neck portion, in particular wherein the average wall thickness of the bottom is higher, in particular about 0,03 mm to 0,2 mm higher, more in particular about 0,05 mm to 0,15 mm higher, most in particular about 0,08 to 0,12 mm higher, for example about 0,1 mm higher, than the average wall thickness of the body portion and/or the neck portion.

According to a sixth embodiment, which can be combined with the first, second and/or third embodiment of this aspect, the overall average wall thickness of the container is maximally 0,8 mm, 0,7 mm, 0,6 mm, 0,55 mm or 0,5 mm or between 0,2 mm and 0,8 mm, more preferably between 0,3 mm and 0,7 mm, most preferably between 0,4 mm and 0,6 mm or between 0,4 mm and 0,55 mm.

According to another aspect of the invention, which can be combined with the previous and subsequent aspects of the invention and vice versa, a primary packaging container, for pharmaceutical, medical, cosmetic, dietetic and/or food products, is provided, which comprises an interior surface with at least one undercut section, wherein at least a portion of the container has a wall thickness with a standard deviation of less than 0,02 mm. Preferably, the standard deviation is lower than 0,015 mm, more preferably lower than 0,01 mm, even more preferably lower than 0,008 mm, 0,007mm, 0,006 mm or 0,005 mm, most preferably lower than 0,0045 mm, 0,004 mm, 0,0035 mm, 0,003 mm, 0,0025 mm, 0,002 mm or 0,0015 mm, for example about 0,001 mm. Additionally or alternatively, the standard deviation is between 0,0001 mm and 0,015 mm, preferably between 0,0002 mm and 0,01 mm, more preferably between 0,0003 mm and 0,05 mm, most preferably between 0,0005 mm and 0,002 mm, for example 0,001 mm. Particularly preferred, the at least one portion is selected from a closed bottom, a body portion extending between the closed bottom and an open top, a neck portion extending between the body portion and the open top, a safety-lock portion extending between the body portion and the neck portion and a shoulder portion extending between the body portion and the neck portion. More preferably, at least two, at least three, at least four or at least five of the previously mentioned portions have a wall thickness with the previously described standard deviation, particularly preferred of between 0,0005 mm and 0,002 mm. It shall be clear that all of the portions of the container according to this aspect of the invention can be realized as described above and below with respect to further aspects of this invention and their embodiments, wherein the features attributed to the respective aspects of the invention may or may not be realized.

According to another aspect of the invention, which can be combined with the previous and subsequent aspects of the invention and vice versa, a primary packaging container, for pharmaceutical, medical, cosmetic, dietetic and/or food products, is provided, which comprises an interior surface with at least one undercut section, wherein at least two, preferably at least three or four, portions selected from a body portion extending between a closed bottom and an open top of the container, a neck portion extending between the body portion and the open top, a safety-lock portion extending between the body portion and the neck portion and a shoulder portion extending between the body portion and the neck portion, have an average wall thickness differing from each other by less than 0,07 mm, preferably by less than 0,06 mm, more preferably by less than 0,05 mm, most preferably by less than 0,04 mm, for example by 0,03 mm. In particular, the average wall thicknesses differ from each other by between 0,005 mm and 0,06 mm, preferably between 0,01 mm and 0,05 mm, more preferably between 0,02 mm and 0,04 mm, most preferably between 0,035 mm and 0,045 mm. More preferably, at least two, at least three or at least four of the previously mentioned portions have an average wall thickness differing from each other by less than 0,07mm, 0,06 mm, 0,05 mm, or 0,04 mm. It shall be clear that all of the portions of the container according to this aspect of the invention can be realized as described above and below with respect to further aspects of this invention and their embodiments, wherein the features attributed to the respective aspects of the invention may or may not be realized.

Preferably, the container according to any of the previously and subsequently described aspects of the invention is produced by a two-step injection molding method. Preferably, injection molding within the meaning of this invention shall mean two-step injection molding. Two step injection molding within the meaning of this invention particularly means injection molding comprising the steps of injecting material into a molding chamber and ejecting the product produced thereby. In particular, two-step injection molding within the meaning of this invention is free of a blowing step, in particular of a step of blowing material from a core member towards a cavity member, in particular between the step of injection and ejection. In particular, two-step injection molding within the meaning of this invention particularly excludes injection blow molding. The two-step injection molding method preferably comprises the following steps:
a) providing a cavity member defining an exterior surface of the container;
b) providing a core member defining an interior surface of the container (1) with at least one undercut section;
c) bringing the cavity member and the core member into a position, in which a molding chamber is formed between the cavity member and the core member;
d) injecting material into the molding chamber to mold the container; and
e) removing the core member (7) from the interior of the container.

Preferably, these steps are conducted as described in detail with respect to the following aspect of the invention, wherein step e) can be realized by means of a collapsible core or not. An example for providing a container with undercut section by the two-step injection molding method without using a collapsible core is for example the use of a sacrificial core. However, preferably, a collapsible core is used as described in detail below.

Producing the container by two-step injection molding, can in particular be identified in the container by its low average wall thickness, low standard deviation and low difference of average wall thickness between different portions of the container. Particularly preferred, the container is produced by an apparatus with collapsible core, in particular as described in detail below, which, compared to using a sacrificial core, is more sustainable. Using a collapsible core can in particular be identified in the interior of the container by thin fins protruding from the interieur surface of the container towards the inside of the container. These fins result from the not completely flat surface of the collapsible core in contact regions of collapsible parts and particularly extend along the longitudinal direction of the collapsible core. However, as these fins are very small, they do not impair the suitability of the container for storing pharmaceutical, medical, cosmetic, dietetic and/or food products therein.

The inventors have surprisingly found that, by using the two-step injection molding method, in particular as described in detail below, the average wall thickness of the container can be lowered from about 0,9 mm (produced by the known three-step injection blow molding method) to an average wall thickness of about 0,45 mm without losing its suitability for pharmaceutical, medical, cosmetic, dietetic and/or food products. Further, the inventors found that using the two-step injection molding method allowed to decrease of standard deviation of the wall thickness within portions of the container, such as the closed bottom, the body portion, the neck portion, shoulder portion and the safety lock means, from 0,02 mm (produced by the known three-step injection blow molding method) to 0,001 mm. In addition, the inventors found that also the uniformity of wall thickness between different portions of the container, such as between the body portion and the shoulder portion, could be increased, i.e. in that the difference of average wall thickness between different portions of the container, such as the shoulder portion and the body portion, could be decreased from about 0,07 mm (produced by the known three-step injection blow molding method) to about 0,03 mm. By further using a collapsible core member for defining the interior surface of the container during injection, in particular as described in detail below, the container can be provided with undercut sections in the interior surface. Thanks to the reduction of the average wall thickness, standard deviation of wall thickness within one portion of the container and difference in wall thickness between different portions of the container, the material consumption could be significantly reduced. Thereby, as melting the material and cooling it before ejecting a container made thereof constitutes the major energy consumption of injection molding, a significant reduction of energy consumption was achieved, thereby increasing the sustainability of the container.

Preferably, the container is designed, in particular in terms of material selection, geometry, manufacturing method and manufacturing apparatus, as explained with respect to the below described aspects of the invention, wherein the features attributed to the respective independent claims may or may not be realized.

The inventors have found that producing the container by a two-step injection molding method using a collapsible core, in particular as described with respect to the following aspects of the invention, instead of the known three-step injection blow molding method, enables producing containers with more uniform wall thickness. Thanks to the more uniform wall thickness, the average wall thickness of the container can be reduced without the risk of partially falling below a minimum wall thickness required for the intended purpose of the invention. Thereby, the material consumption and thus also the energy consumption for melting the material can be reduced, thereby solving the object of enhanced sustainability of such containers.

According to another aspect of the invention, which can be combined with the previous aspects as well as features and embodiments described in combination therewith, and vice versa, a container, in particular a primary packaging container, for pharmaceutical, medical, cosmetic, dietetic and/or food products, is provided, which is injection molded by a method comprising the following steps:
a) providing a cavity member defining an exterior surface of the container;
b) providing a core member defining an interior surface of the container with at least one undercut section;
c) bringing the cavity member and the core member into a position, in which a molding chamber is formed between the cavity member and the core member;
d) injecting material into the molding chamber to mold the container; and
e) removing the core member from the interior of the container, whereby the core member collapses for passing the undercut region.

The core member used within this aspect of the invention and the below described aspects of the invention can be referred to as collapsible core. By using a collapsible core, containers having an interior surface with undercut section can be produced in that the core member delimits the interieur of the molding chamber in step c) with a core surface having an undercut section so that, by injecting the material in step d), a container assuming the surface of the core member, i.e. an interieur surface with undercut section, is formed, wherein the core can be removed from the interieur of the container by collapsing it so that it can pass the undercut region. The inventors have found that the two-step injection molding method can be used to produce containers, in particular a primary packaging container, for pharmaceutical, medical, cosmetic, dietetic and/or food products, in particular containers having a volumetric capacity and/or an undercut ratio within the above defined ranges and/or meeting the above described requirements for such containers. In addition, the inventors found that, compared to the three-step injection blow molding method, the energy consumption for producing such containers can be significantly reduced by using the described two-step injection molding method with collapsible core, thereby increasing the sustainability of the container. Further to that, the inventors found hat, compared to the three-step injection blow molding method, the two-step injection molding method with collapsible core provides containers with a smaller standard deviation of the wall thickness thereby allowing to reduce the wall thickness of the container thus reducing the overall material consumption so that the sustainability of the container is further increased. Further, by omitting the blowing step of the three-step injection blow molding method, cycle times can be reduced. The reduction of cycle times and of material consumption further decreases the required energy demand for melting the material and keeping it hot during manufacturing thus further reducing the energy consumption so that the sustainability of the container is further increased. In particular, the inventors found that by means of the present invention, in particular by using the two-step injection molding method with collapsible core, the material consumption can be reduced by 50% and the energy consumption can be reduced by 86% compared to the three-step injection blow molding method.

In particular, the molding chamber has the shape of the later container. As described above and below, a container within the meaning of the present invention preferably extends along a longitudinal axis and comprises a closed bottom at one end of a longitudinal axis and an open top at the opposite end of the longitudinal axis. Respectively, the molding chamber formed in step c) preferably extends along the longitudinal axis of the later container and defines the closed bottom of the later container at one end of the longitudinal axis and an open top of the later container at the opposite end of the longitudinal axis.

In particular, in step a) a cavity member is provided which defines the exterior surface of the container in that, in step c), the cavity member defines an exterior surface of the molding chamber.

Preferably, the exterior surface of the molding chamber in step c) comprises an in particular at least partially cylindrically shaped exterior jacket surrounding the longitudinal axis in circumferential direction and extending along the longitudinal axis from the closed bottom to the open top.

The exterior jacket preferably comprises at least two cylindrical sections, in particular one forming a body portion of the later container and one forming a neck portion of the later container, wherein at least one of the sections, preferably the section forming the neck portion, preferably comprises recesses with a thread form to provide the exterior of the container with a thread. Preferably the at least two cylindrical sections have a different diameter, wherein the cylindrical section forming the later body portion has a larger diameter than the cylindrical section forming the later neck portion. More preferably, the exterior jacket comprises at least three cylindrical sections, in particular the two cylindrical sections forming the body portion and the neck portion of the later container and a third cylindrical section. The third cylindrical section can be a cylindrical section forming a safety-lock portion of the later container, wherein the cylindrical section forming the safety-lock portion preferably comprises recesses with the form of safety lock means for providing the exterior of the container with safety lock means and is preferably located in between the cylindrical sections forming the later body and neck portion. Alternative, the third cylindrical section can be a cylindrical section forming a mouth portion of the later container, wherein the cylindrical section forming the later mouth portion is preferably located between the open top and the section forming the later neck portion. Preferably the at least three cylindrical sections have a different diameter, wherein the cylindrical section forming the later body portion has a larger diameter than the cylindrical section forming the later neck portion and preferably wherein, if the third cylindrical portion is the section forming the later safety-lock portion, it has a smaller diameter than the section forming the later body portion and/or a larger diameter than the section forming the later neck portion, or, if the third cylindrical section is the section forming the later mouth portion, it has a smaller diameter than section forming the later neck portion. Even more preferably, the exterior jacket comprises at least four cylindrical sections, in particular the two cylindrical sections forming the body portion and the neck portion of the later container and a third and a fourth cylindrical section, wherein preferably the third cylindrical section is the previously described cylindrical section forming the safety-lock portion of the later container and the fourth cylindrical section is the cylindrical section forming the mouth portion of the later container. Preferably the at least four cylindrical sections have a different diameter, preferably wherein the cylindrical section forming the later body portion has a larger diameter than the cylindrical section forming the safety-lock portion, and wherein the cylindrical section forming the later safety-lock portion has a larger diameter than the cylindrical section forming the later neck portion, and wherein the cylindrical section forming the later neck portion has a larger diameter than the cylindrical section forming the later mouth portion.

In addition to the exterior jacket, the exterior surface of the molding chamber in step c) further comprises an in particular disc-shaped exterior bottom surface defining the exterior surface of the closed bottom of the later container. Preferably, the cavity member comprises an injection inlet for injection material into the molding chamber. Most preferably, the injection inlet is realized in the exterior bottom surface of the exterior surface of the molding chamber.

In addition to the exterior jacket and the exterior bottom surface, the exterior surface of the molding chamber in step c) preferably further comprises an in particular ring-shaped exterior top surface surrounding the open top of the later container in circumferential direction of the longitudinal axis and connecting the exterior jacket of the exterior surface of the molding chamber with the below described interior surface, in particular with the below described interieur jacket, of the molding chamber, in particular to close the molding chamber at the open top, in particular in that, beside the above described injection inlet, the molding chamber is concealed, in particular in a fluid-tight manner. In this regard, fluid-tight particularly means fluid-tight with respect to molten material, in particular molten polymer, more in particular molten polyolefin, most in particular molten polypropylene, being injected with a common injection pressure used in the art for injection molding containers, in particular with the above defined dimensions, wall thicknesses and/or recesses.

For bringing the cavity member in the position of step c) and for ejecting the container after step d), in particular in step e), the cavity member can be made of multiple parts being movable, in particular relative, to each other. In particular, parts of the cavity member can be movable, in particular relative to each other, parallel to the longitudinal axis and/or orthogonal to the longitudinal axis.

In particular, in step b) a core member is provided which defines the interieur surface of the container with at least one undercut section in that, in step c), the core member defines an interieur surface of the molding chamber.

Preferably, the interieur surface of the molding chamber in step c) comprises an in particular at least partially cylindrically shaped interieur jacket surrounding the longitudinal axis in circumferential direction and extending along the longitudinal axis from the closed bottom at least to the open top. Preferably, the interieur jacket extends in step c) parallel to the exterior jacket, wherein the interieur and exterior jackets are preferably spaced from each other by the wall thickness of the later container, in particular by between 0,2 mm and 1,00 mm, preferably between 0,3 mm and 0,8 mm, more preferably between 0,4 mm and 0,6 mm, most preferably 0,5 mm.

The interieur jacket preferably comprises at least two cylindrical sections, in particular one forming a body portion of the later container and one forming a neck portion of the later container, preferably wherein at least one section is, preferably both sections are, free of recesses, protrusions and/or threads. Preferably the at least two cylindrical sections have a different diameter, wherein the cylindrical section forming the later body portion has a larger diameter than the cylindrical section forming the later neck portion. More preferably, the interieur jacket comprises at least three cylindrical sections, in particular the two cylindrical sections forming the body portion and the neck portion of the later container and a third cylindrical section. The third cylindrical section can be a cylindrical section forming a safety-lock portion of the later container, preferably wherein the cylindrical section forming the safety-lock portion is free of recesses, protrusions and/or threads and/or is located in between the cylindrical sections forming the later body and neck portion. Alternative, the third cylindrical section can be a cylindrical section forming a mouth portion of the later container, wherein the cylindrical section forming the later mouth portion is preferably located between the open top and the section forming the later neck portion. Preferably the at least three cylindrical sections have a different diameter, wherein the cylindrical section forming the later body portion has a larger diameter than the cylindrical section forming the later neck portion and preferably wherein, if the third cylindrical portion is the section forming the later safety-lock portion, it has a smaller diameter than the section forming the later body portion and/or a larger diameter than the section forming the later neck portion, or, if the third cylindrical section is the section forming the later mouth portion, it has a smaller diameter than section forming the later the neck portion. Even more preferably, the interieur jacket comprises at least four cylindrical sections, in particular the two cylindrical sections forming the body portion and the neck portion of the later container and a third and a fourth cylindrical section, wherein preferably the third cylindrical section is the previously described cylindrical section forming the safety-lock portion of the later container and the fourth cylindrical section is the cylindrical section forming the mouth portion of the later container. Preferably the at least four cylindrical sections have a different diameter, preferably wherein the cylindrical section forming the later body portion has a larger diameter than the cylindrical section forming the safety-lock portion, and wherein the cylindrical section forming the later safety-lock portion has a larger diameter than the cylindrical section forming the later neck portion, and wherein the cylindrical section forming the later neck portion has a larger diameter than the cylindrical section forming the later mouth portion.

In addition to the interieur jacket, the interieur surface of the molding chamber in step c) further comprises an in particular disc-shaped interieur bottom surface defining the interieur surface of the closed bottom of the later container. Preferably, interieur bottom surface extends in step c) parallel to the exterior bottom surface, wherein the interieur and exterior bottom surface are preferably spaced from each other by the wall thickness of the later container, in particular by between 0,2 mm and 1,00 mm, preferably between 0,3 mm and 0,8 mm, more preferably between 0,4 mm and 0,6 mm, most preferably 0,5 mm.

In particular for bringing the core member in the position of step c) and for ejecting the container after step d), in particular in step e), the core member is designed as a collapsible core. The collapsible core can be designed to adopt at least two operation states, namely a collapsed state and an expanded state. In the collapsed state, the collapsible core particularly has a smaller radial extension than in the expanded state, in particular such a small radial extension that, in particular in step e), it can pass the undercut section, in particular in opening direction, so that it can be removed from the interior of the container, in particular through the open top of the container. In particular in step c), the collapsible core is in its expanded state, in which it preferably has the above and below described geometry. In particular in the expanded state, the collapsible core can comprise a central portion, rod portions, in particular four rod portions, radially extending from the central portion, and cylinder segment portions, in particular four cylinder segment portions, extending between the rod portions. The cylinder segment portions and the rod portions preferably form together the previously described interieur jacket. At their front face, the central portion, the rod portions and the cylinder segment portions preferably form the previously described disc shaped interieur bottom surface. The central portion can be mounted slidably relative to the rod portions in that, by removing the central portion in step e) in opening direction, the rod portions tilt in radial direction inwards thereby reducing their radial extension, in particular the part of the interieur jacket formed by them, so that they can pass the undercut section and thus be removed from the container in opening direction through the open top of the container. The rod portions can additionally be mounted slidably relative to the cylinder segment portions in that, by removing the rod portions in step e) in opening direction, the cylinder segment portions tilt in radial direction inwards thereby reducing their radial extension, in particular the part of the interieur jacket formed by them, so that they can pass the undercut section and thus be removed from the container in opening direction through the open top of the container.

The position of step c), in which a molding chamber is formed between the cavity member and the core member, can be referred to as injection position. For bringing the core member and the cavity member into the injection position, the core member and the cavity member can be brought simultaneously or successively into the injection position. Preferably, the cavity member and the core member are brought successively into the injection position. Even more preferably, the cavity member is brought before the core member into the injection position.

For bringing the core member into the injection position, the core member is preferably shifted, in particular along the longitudinal axis, more in particular in opposite to the opening direction, into the injection position, where the core member is in expanded state. For this purpose, the central portion of the cylinder segment portions are preferably first shifted towards the injection position, in particular being tilted in radial direction inwards. In a second step, the rod portions are preferably shifted towards the injection position, in particular being tilted in radial direction inwards, thereby expanding the cylinder segment portions in radial direction outwards. In a third step the central portion is preferably shifted towards the injection position thereby expanding the cylinder segment portions and the rod portions in radial direction outwards so that, in particular after the third step, the core member adopts its expanded state in the injection position.

For bringing the cavity member into the injection position, the cavity member is preferably moved in radial direction towards the longitudinal axis and/or in axial direction towards the later molding chamber, in particular towards the position of the core member in the injection position. For this purpose, the cavity member can comprise cavity parts being movable along the longitudinal axis and/or cavity parts being movable in radial direction. A part being movable along the longitudinal axis preferably provides the previously described exterior surface sections for the formation of the body portion and closed bottom of the later container and/or the previously described ring-shaped exterior top surface of the molding chamber. Additionally or alternatively, the cavity member comprises at least two parts being movable in radial direction The two parts being movable in radial direction particularly provide the exterior surface section for the formation of the previously described neck and/or safety lock portion of the later container and/or the previously described thread and/or safety lock means on the exterior surface, in particular on the neck and/or safety-lock portion of the of the later container.

Particularly preferred the cavity member comprises at least one part being movable in axial direction and at least two parts being movable in radial direction, in particular wherein the two parts being movable in radial direction provide the exterior surface section for the formation of the previously described neck and/or safety lock portion of the later container, and wherein one part being movable in axial direction provides the previously described exterior surface sections for the formation of the body portion and closed bottom of the later container or the previously described ring-shaped exterior top surface of the molding chamber. Even more preferably, the cavity member comprises at least two parts being movable in axial direction and at least two parts being movable in radial direction, in particular wherein the two parts being movable in radial direction provide the exterior surface section for the formation of the previously described neck and/or safety-lock portion of the later container, and wherein one part being movable in axial direction provides the previously described exterior surface sections for the formation of the body portion and closed bottom of the later container, and wherein one part being movable in axial direction provides the previously described ring-shaped exterior top surface of the molding chamber.

For injecting material into the molding chamber in step d), the cavity member and/or the core member can comprise an injection inlet for injection material into the molding chamber. Preferably, the injection inlet is realized in the core member, more preferably in the exterior bottom surface of the exterior surface of the molding chamber. For injecting the material, an injection nozzle can be used, which can be positioned at the injection inlet. Prior to injection, the material can be melted, for example by a screw extruder.

For removing the core member form the interior of the container in step e), whereby the core member collapses for passing the undercut region, the core member can be removed as described above, in particular in the reverse direction as previously described with respect to step c) for bringing the core member into the injection position. In addition to the core member, the cavity member can be removed from the container in step e), preferably as described above, in particular in the reverse direction as previously described with respect to step c) for bringing the cavity member into the injection position.

In the following, preferred embodiments of the inventive container according to any of the previous and below described aspects of the invention will be described.

In a seventh embodiment, the interior of the container is at least partially rotationally shaped along a longitudinal axis and comprises a closed bottom at one end of the longitudinal axis and an open top at the opposite end of the longitudinal axis. Preferably the closed bottom is circular shaped and particularly extends orthogonal to the longitudinal axis. The open top is preferably circular shaped, in particular is a circular opening, extending preferably orthogonal to the longitudinal axis and/or parallel to the closed bottom.

In a eighth embodiment, preferably according to the seventh embodiment, the undercut section is located between the smallest diameter of the interior and the largest diameter of the interior between the smallest diameter and the bottom. Preferably, the smallest diameter is located in a top region extending from the open top towards the bottom along 30%, more preferably along 15%, even more preferably along 5%, most preferably along 2.5% of the axial extension of the container. Particularly preferred, the smallest diameter is located at the open top of the container, and is in particular delimited by the above and below described mouth portion of the container. The largest diameter of the interieur between the smallest diameter and the closed bottom is preferably located in a bottom region extending from the closed bottom towards the open top along 70%, preferably along 50%, more preferably along 30%, even more preferably along 20%, most preferably along 10 %, of the axial extension of the container. Particularly preferred, the largest diameter is located at the closed bottom of the container, and is in particular delimited by the above and below described body portion of the container. In particular, the largest diameter is larger, preferably between 2 mm and 20 mm larger, more preferably between 3 mm and 15 mm larger, even more preferably between 4 mm and 12 mm larger, most preferably between 5 mm and 9 mm larger than the smallest diameter. In particular, the smallest diameter is between 20 mm and 50 mm, more preferably between 25 mm and 45 mm, even more preferably between 30 mm and 35 mm, for example 32,4 mm. Additionally or alternatively, the largest diameter is in particular between 20 mm and 90 mm, preferably between 30 mm and 60 mm, more preferably between 35 mm and 45 mm, even more preferably between 37.5 mm and 42.5 mm, for example 39,4 mm.

In addition to the above-described undercut ratio, the following part of the description will describe specific dimensions, undercut ratios, and diameter ratios being beneficial for containers for pharmaceutical, medical, cosmetic, dietetic and/or food products.

Preferably, the largest diameter divided by the smallest diameter defines an undercut ratio of at least 1.05, preferably at least 1.10, more preferably at least 1.15, most preferably of at least 1.20. If the largest diameter is for example 39.4 cm and the smallest diameter is 32,4 mm, the container has an undercut ratio of 1.216.

The present invention is intended to cover, for example, containers comprising only one undercut section, for example containers having only two cylindrical portions, such as a body portion and a neck portion, wherein the neck portion has the smallest diameter and the body portion has the largest diameter, so that the undercut ratio of the container is equal to a body/neck ratio being calculated by dividing the diameter of the body portion by the diameter of the neck portion. However, as described with respect to the following embodiments, the present invention also intends to cover containers with more than one undercut section. In particular in such cases, the overall undercut ratio of the container can be larger than the ratio of two parts between which an undercut section is formed.

In a ninth embodiment, preferably according to any of the to seventh the eighth embodiment, the container comprises a body portion extending from the closed bottom towards the open top and a neck portion extending between the body portion and the open top, wherein the inside of the body portion and the neck portion is preferably cylindrically shaped.

Preferably, the body portion extends from the closed bottom towards the open top along between 20% and 90%, preferably between 30% and 80%, more preferably between 40 % and 70%, most preferably between 50% and 60 %, of the axial extension of the container. Particularly preferred, the body portion comprises a cylindrical jacket extending from the circular bottom towards the open top, in particular wherein the cylindrical jacket and the circular bottom together have the shape of a cup. The cylindrical jacket of the body portion preferably has the shape of an, in particular perfect, hollow cylinder with an inside diameter and an outside diameter being separated by each other by an, in particular constant, wall thickness. As described in detail above and below, the inside surface, and preferably also the outside surface, of the body portion is preferably free of any recesses, protrusions and/or threads. Preferably, the cylindrical jacket of the body portion has an inside diameter between 20 mm and 90 mm, preferably between 30 mm and 60 mm, more preferably between 35 mm and 45 mm, even more preferably between 37.5 mm and 42.5 mm, for example 39,4 mm and/or extends along the longitudinal axis between 10 mm and 60 mm, preferably between 15 mm and 45 mm, more preferably between 20 mm and 35 mm, most preferably between 25 mm and 30 mm, for example along 27 mm. Preferably, the circular bottom and the cylindrical jacket of the body portion have a wall thickness between 0,2 mm and 1,0 mm, preferably between 0,3 mm and 0,8 mm, more preferably between 0,4 mm and 0,6 mm, most preferably of 0,5 mm. The circular bottom preferably has an inside diameter and/or a wall thickness in the same range, in particular with substantially the same value, as the cylindrical jacket. Substantially the same value within the meaning of the present invention with respect to a diameter is in particular to be understood as encompassing a variation of up to 5%, 3%, 2% or 1%. Substantially the same value within the meaning of the present invention with respect to a wall thickness is in particular to be understood as encompassing a difference of less than 0,15mm, preferably less than 0,07 mm, more preferably less than 0,06 mm, most preferably preferably less than 0,05 mm.

Additionally or alternatively, the neck portion extends between the body portion and the open top along between 10% and 50%, preferably between 15% and 40%, more preferably between 20 % and 35%, most preferably between 25% and 30%, of the axial extension of the container. Particularly preferred, the neck portion comprises a cylindrical jacket in particular having the shape of an, in particular perfect, hollow cylinder with an inside diameter and an outside diameter being separated by each other by an, in particular constant, wall thickness. As described in detail above and below, the neck portion can comprise thread means on the outside surface of the cylindrical jacket, wherein the inside surface is preferably free of any recesses, protrusions and/or threads. Preferably, the cylindrical jacket of the neck portion has an inside diameter between 10 mm and 55 mm, preferably between 20 mm and 45 mm, more preferably between 25 mm and 40 mm, even more preferably between 30 mm and 35 mm, for example of 33 mm and/or extends along the longitudinal axis between 5 mm and 35 mm, preferably between 7,5 mm and 25 mm, more preferably between 10 mm and 20 mm, most preferably between 12,5 mm and 17,5 mm, for example along 14,4 mm. Preferably, the circular jacket of the neck portion has a wall thickness between 0,2 mm and 1,0 mm, preferably between 0,3 mm and 0,8 mm, more preferably between 0,4 mm and 0,6 mm, most preferably of 0,5 mm. The cylindrical jacket of the neck portion preferably has a wall thickness in the same range, in particular with substantially the same value, as the cylindrical jacket and the circular bottom of the body portion.

Preferably, the diameter of the neck portion divided by the diameter of the body portion defines a body/neck ratio of at least 1.04, preferably of at least 1.09, more preferably at least 1.13, most preferably of at least 1.18. For example, if the cylindrical jacket of the neck portion has an inside diameter of 33 mm while the cylindrical jacket of the body portion has an inside diameter of 39,4 mm, the body/neck ratio is 1.194.

In a tenth embodiment, preferably according to any of the seventh to the ninth embodiment, the container further comprises a mouth portion extending between the open top and the neck portion, wherein the inside of the mouth portion is preferably cylindrically shaped. Preferably, the mouth portion extends between the open top and the neck portion along between 0,5 % and 10%, preferably between 1,0% and 5%, more preferably between 1,5% and 2,5%, most preferably between 1,75% and 2,25%, of the axial extension of the container. Particularly preferred, the mouth portion comprises cylindrical jacket in particular having the shape of an, in particular perfect, hollow cylinder with an inside diameter and an outside diameter being separated by each other by an, in particular constant, wall thickness. As described in detail above and below, the mouth inside surface, and preferably the outside surface, of the mouth portion is preferably free of any recesses, protrusions and/or threads. Preferably, the cylindrical jacket of the mouth portion has an inside diameter between 10 mm and 55 mm, preferably between 20 mm and 45 mm, more preferably between 25 mm and 40 mm, even more preferably between 30 mm and 35 mm, for example of 32,4 mm and/or extends along the longitudinal axis between 0,2 mm and 10 mm, preferably between 0,4 mm and 5 mm, more preferably between 0,6 mm and 2,5 mm, most preferably between 0,8 mm and 1,5 mm, for example along 1 mm. Preferably, the circular jacket of the mouth portion has a wall thickness between 0,2 mm and 1,0 mm, preferably between 0,3 mm and 0,8 mm, more preferably between 0,4 mm and 0,6 mm, most preferably of 0,5 mm. The cylindrical jacket of the mouth portion preferably has a wall thickness in the same range, in particular with substantially the same value, as the cylindrical jacket and the circular bottom of the body portion and/or as the cylindrical jacket of the neck portion.

Preferably, the diameter of the body portion divided by the diameter of the mouth portion defines a body/mouth ratio of at least 1.05, preferably of at least 1.10, more preferably at least 1.15, most preferably of at least 1.20, and/or wherein the diameter of the neck portion dived by the diameter of the mouth portion defines a neck/mouth ratio of more than 1, preferably of at least 1.01, more preferably of at least 1.015. For example, if the cylindrical jacket of the mouth portion has an inside diameter of 32,4 mm and if the cylindrical jacket of the body portion has an inside diameter of 39,4 mm the body/mouth ratio is 1,216, i.e. equal to the above exemplarily calculated undercut ratio. In particular, the undercut ratio of the container is equal to the body/mouth ratio. Further, if for example the cylindrical jacket of the mouth portion has an inside diameter of 32,4 mm and if the cylindrical jacket of the neck portion has an inside diameter of 33 mm, the neck/mouth ratio is 1,019.

In a eleventh embodiment, preferably according to any of the seventh to the tenth embodiment, the container further comprises a safety-lock portion extending between the neck portion and the body portion, wherein the inside of the safety-lock portion is preferably cylindrically shaped. Preferably, the safety-lock portion extends between the neck portion and the body portion along between 5 % and 30%, preferably between 7,5 % and 25 %, more preferably between 10% and 20%, most preferably between 12,5 % and 17,5%, of the axial extension of the container. Particularly preferred, the safety-lock portion comprises cylindrical jacket in particular having the shape of an, in particular perfect, hollow cylinder with an inside diameter and an outside diameter being separated by each other by an, in particular constant, wall thickness. As described in detail above and below, the interieur surface of the safety-lock portion is preferably free of any recesses, protrusions and/or threads, wherein its exterior surface preferably comprises safety lock means. Preferably, the cylindrical jacket of the safety-lock portion has an inside diameter between 20 mm and 90 mm, preferably between 30 mm and 60 mm, more preferably between 35 mm and 45 mm, even more preferably between 37.5 mm and 42.5 mm, for example of 38 mm and/or extends along the longitudinal axis between 2 mm and 30 mm, preferably between 4 mm and 20 mm, more preferably between 5 mm and 15 mm, most preferably between 6 mm and 10 mm, for example along 8 mm. Preferably, the circular jacket of the safety-lock portion has a wall thickness between 0,2 mm and 1,0 mm, preferably between 0,3 mm and 0,8 mm, more preferably between 0,4 mm and 0,6 mm, most preferably of 0,5 mm. The cylindrical jacket of the safety-lock portion preferably has a wall thickness in the same range, in particular with substantially the same value, as the cylindrical jacket and the circular bottom of the body portion, as the cylindrical jacket of the neck portion and/or as the cylindrical jacket of the mouth portion.

Preferably, the diameter of the safety-lock portion divided by the diameter of the neck portion defines a safety-lock/neck ratio of more than 1, preferably of more than 1.04, more preferably of more than 1.08, most preferably of more than 1.12, and/or wherein the diameter of the body portion dived by the diameter of the safety-lock portion defines a body/safety-lock ratio of more than 1, preferably of at least 1.02, more preferably of at least 1.03. For example, if the cylindrical jacket of the safety-lock portion has an inside diameter of 38 mm and if the cylindrical jacket of the neck portion has an inside diameter of 33 mm the safety-lock/neck ratio is 1,152. Further, if for example the cylindrical jacket of safety-lock portion has an inside diameter of 38 and if the cylindrical jacket of the body portion has an inside diameter of 39,4 mm, the body/safety-lock ratio is 1,037.

In a twelfth embodiment, preferably according to any of the seventh to the eleventh embodiment, the container further comprises a shoulder portion extending between the neck portion and the body portion, wherein the inside of the shoulder portion has preferably a truncated cone shape tapering towards the top. Preferably, the inside surface of the shoulder portion is free of any recesses, protrusions and/or threads, wherein its exterior surface of the shoulder portion preferably comprises at least one, preferably at least two, in particular ring-shaped protrusion protruding in radial direction beyond the exterior surface of the body portion and/or of the safety-lock portion.

Preferably, the shoulder portion is separated into two parts by the safety-lock portion, in particular the safety-lock portion according to the eleventh embodiment, in particular in that a lower part of the shoulder portion facing the closed bottom connects the body portion with the safety-lock portion while an upper part of the shoulder portion facing the open top connects the safety-lock portion with the neck portion. In this case, the exterior surface of the shoulder portion preferably comprises two of the previously described, in particular ring-shaped, protrusions preferably being spaced from each other in axial direction, in particular being spaced in axial direction by the safety-lock portion and preferably providing the safety-lock portion with an upper and a lower rim for guiding safety lock means of a closure being intended to interact with safety lock means of the safety-lock portion.

In a thirteenth embodiment, preferably according to any of the seventh to the twelfth embodiment, the at least one undercut section is realized by a portion between the neck portion and the mouth portion according to the tenth embodiment and/or by the shoulder portion according to twelfth embodiment, preferably wherein the container comprises at least two undercut sections being preferably realized by a portion between the neck portion and the mouth portion and at least part of the shoulder portion or by an upper and lower part of the shoulder portion, more preferably wherein the container comprises at least three undercut sections being realized by a portion between the neck portion and the mouth portion, the upper part of the shoulder portion and the lower part of the shoulder portion.

Preferably, the container is designed in that the cross section of the container is reduced towards the open top. Particularly preferred, the container comprises at least two portions, preferably the body portion and the neck portion, with constant, in particular circular, cross section along the longitudinal axis and at least one section, preferably the shoulder portion, with a cross section tapering, in particular with truncated cone shape, towards the open top. More preferably, the container comprises at least three portions, preferably the body portion, the neck portion and a portion selected from the mouth portion and the safety-lock portion, with constant, in particular circular, cross section along the longitudinal axis and at least two portions, preferably the upper and lower part of the shoulder portion or at least a part of the shoulder portion and a portion between the mouth and neck portion, with a cross section tapering, in particular with truncated cone shape, towards the open top. Most preferably, the container comprises at least four portions, preferably the body portion, the neck portion, the mouth portion and the safety-lock portion, with constant, in particular circular, cross section along the longitudinal axis and at least three portions, preferably the upper and lower part of the shoulder portion and a portion between the mouth and neck portion, with a cross section tapering, in particular with truncated cone shape, towards the open top.

In a fourteenth embodiment, preferably according to any of the seventh to the thirteenth embodiment, the wall thickness of the body portion deviates from the wall thickness of the neck portion and/or shoulder portion by less than 0,07 mm, preferably by less than 0,06 mm, more preferably by less than 0,5 mm, most preferably by less than 0,4 mm.

In a fifteenth embodiment, preferably according to any of the seventh to the fourteenth embodiment, except of diameter steps and/or truncated parts reducing the diameter of the interior towards the opening, preferably according to first to the thirteenth embodiment, the interior of the container is free of recesses, protrusions and/or threads, in particular of any protrusions. Thereby, the interior surface of the container is realized relatively flat, thereby reducing the risk that particles and endotoxin adhere to the interieur surface, thereby enhancing the suitability of the container for pharmaceutical, medical, cosmetic, dietetic and/or food products.

In a sixteenth embodiment, preferably according to any of the seventh to the fifteenth embodiment, the exterior of the container comprises thread means, in particular at the exterior of the neck portion, in particular according to the ninth embodiment, for a threaded closure, and/or safety lock means, in particular at the exterior of the safety-lock portion, in particular according to the eleventh embodiment, for preventing children from removing a closure from the container.

In an seventeenth embodiment, preferably according to any of the seventh to the sixteenth embodiment, the wall thickness of the container, in particular according to any of the third to fourteenth embodiment and/or except of regions with thread means and/or safety lock means according to the sixteenth embodiment, is between 0,2 mm and 1,00 mm, preferably between 0,3 mm and 0,8 mm, more preferably between 0,4 mm and 0,6 mm, most preferably 0,5 mm.

In a eighteenth embodiment, preferably according to any of the seventh to the seventeenth embodiment, the container comprises a polymer, preferably a Polyolefin, more preferably a Polyolefin selected from Polypropylene, Polyethylene, Cyclo-Olefin Polymer and/or Cyclo-Olefin Co-Polymer. The inventors found that using a polymer instead of glass for the container enables reducing energy consumption for its production, for instance in that polymers can be molten with less energy, but also in that glass containers have larger tolerances thus requiring a higher wall thickness which raises energy and material consumption. Further, glass container are more susceptible to breakage thus requiring complex handling apparatuses during the entire life time from manufacturing of the container over its transportation and filling to its use by the customer. The problem of breakage can be partially solved by hardening the container which however comes again with an increased energy consumption. Thus, by using polymers instead of glass, the material and energy consumption can be reduced leading to an improved sustainability. In particular polyolefins, more in particular Polypropylene, Polyethylene, Cyclo-Olefin Polymer and/or Cyclo-Olefin Co-Polymer, have been found to be particularly suitable for the intended use of the container, in particular for pharmaceutical, medical, cosmetic, dietetic and/or food products.

In a nineteenth embodiment, preferably according to the eighteenth embodiment, the polymer is Polypropylene. The inventors have found that the use of polypropylene is particularly advantageous for the production of containers for pharmaceutical, medical, cosmetic, dietetic and/or food products. In particular, the inventors found that, compared to polyethylene, polypropylene is harder, stiffer, firmer, more resistant against heat and most chemicals used for pharmaceutical, medical, cosmetic, dietetic and/or food products, and has a lower density. Further, the inventors found that polyethylene is disadvantageous over polypropylene in that polyethylene gets faster brittle when being subjected to sun, is less able to be labeled by adhesive means, attracts more electrical charges, and softens already at 80°C, while polypropylene softens at 100°C. In particular in view of the above listed advantages of polypropylene, the inventors found that using polypropylene instead of polyethylene allows manufacturing containers for pharmaceutical, medical, cosmetic, dietetic and/or food products, purely out of polypropylene thereby allowing to manufacture the containers with less energy consumption and thereby more sustainable. Further, thanks to the use of only one material, recycling the container is easier thereby making it again more sustainable.

In a twentieth embodiment, preferably according to eighteenth or nineteenth embodiment, the container consists of the polymer. In other words, according to this embodiment, the container is made to 100 % of a polymer, preferably of a polyolefins, more preferably of a polymer selected from a Polypropylene, Polyethylene, Cyclo-Olefin Polymer and/or Cyclo-Olefin Co-Polymer, most preferably of Polypropylene. As described above, thereby, the sustainability of the container with respect to manufacturing and recycling can be increased.

Further, the invention relates to a packaging product, in particular a primary packaging product, for pharmaceutical, medical, cosmetic, dietetic and/or food products, comprising a container, in particular according to any of the previously and subsequently described aspects and/or their embodiments, and a closure for sealing the container after being filled with the product to be stored. Preferably, the container and the closure are made from the same material, wherein the material preferably is a polymer, more preferably a polyolefin, even more preferably a polymer selected from Polypropylene, Polyethylene, Cyclo-Olefin Polymer and/or Cyclo-Olefin Co-Polymer, most preferably a Polypropylene. Particularly preferred, the closure is a screw or a snap cap. Particularly preferred, the closure, in particular cap, provides a desiccant function, in particular desiccant means. Preferably, the packaging product is free of a handle. In particular, the packaging product can be a prefillable or prefilled packaging product, in particular primary packaging product.

According to another aspect of the invention, which can be combined with the previous aspects as well as features and embodiments described in combination therewith, and vice versa, a method for injection molding of a container, in particular a primary packaging container, for pharmaceutical, medical, cosmetic, dietetic and/or food products, is provided, wherein the method comprises the following steps:
a) providing a cavity member defining an exterior surface of the container;
b) providing a core member defining an interior surface of the container with at least one undercut section;
c) bringing the cavity member and the core member into a position, in which a molding chamber is formed between the cavity member and the core member;
d) injecting material into the molding chamber to mold the container; and
e) removing the core member from the interior of the container by collapsing the core member to allow the core member to pass the undercut section.

The method, in particular at least one, two, three, four or five of the steps a) to e) can be conducted as previously described with respect to the inventive container. In particular, the container can be realized in that it can be produced by the inventive method.

According to another aspect of the invention, which can be combined with the previous aspects as well as features and embodiments described in combination therewith, and vice versa, an apparatus for injection molding of a container, in particular a primary packaging container, for pharmaceutical, medical, cosmetic, dietetic and/or food products is provided, wherein the apparatus comprises:
a) a cavity member defining an exterior surface of the container;
b) a core member defining an interior surface of the container with at least one undercut section;
c) a mechanism for bringing the cavity member and the core member into a position, in which a molding chamber is formed between the cavity member and the core member; and
d) an injection member for injecting material into the molding chamber to mold the container;
wherein the mechanism is adapted to remove core member from the interior of the container by collapsing the core member to allow the core member to pass the undercut section.

The apparatus can be realized in that it can conduct the method, in particular at least one, two, three, four or five of the steps a) to e), as previously described with respect to the inventive container and/or method. In particular, the container can be realized in that it can be produced by the inventive apparatus. In particular, the mechanism can be realized in that it can operate, in particular move, the previously described parts, such as the cavity member, the core member, the injection means etc., in particular as described above.

Preferred embodiments are given in the dependent claims.

In the following, further properties, features and advantages of the invention will become apparent by means of a description of preferred embodiments of the invention with reference to the accompanying exemplary drawings, in which show:
- Figure 1: a side view of an inventive container having an interieur surface with at least one undercut section;
- Figure 2: a cross section view of the container shown in figure 1 along cutting line A-A;
- Figure 3: a cross section view of an apparatus for injection molding the container of Figure 1, shown in injection position;
- Figure 4: a schematically indicated part of the process of injection molding an inventive container by bringing the core member into the injection position of step c), injecting the material in step d) and removing the core member from the container in step e);
- Figure 5: a detailed cross section view of the core member of figure 3 in injection position;
- Figure 6: a detailed cross section view of the core member of figure 3 at the beginning of collapsing it for passing the undercut section in step e); and
- Figure 7: a detailed cross section view of the core member of figure 3 in the middle of collapsing it for passing the undercut section in step e).

In the following description of exemplary embodiments, a container is referred to with reference sign 1, an apparatus for injection molding the container is referred to with reference sign 3, a cavity member is referred to with reference sign 5 and a core member is referred to with reference sign 7. The same or similar features are referred to with the same or similar reference signs.

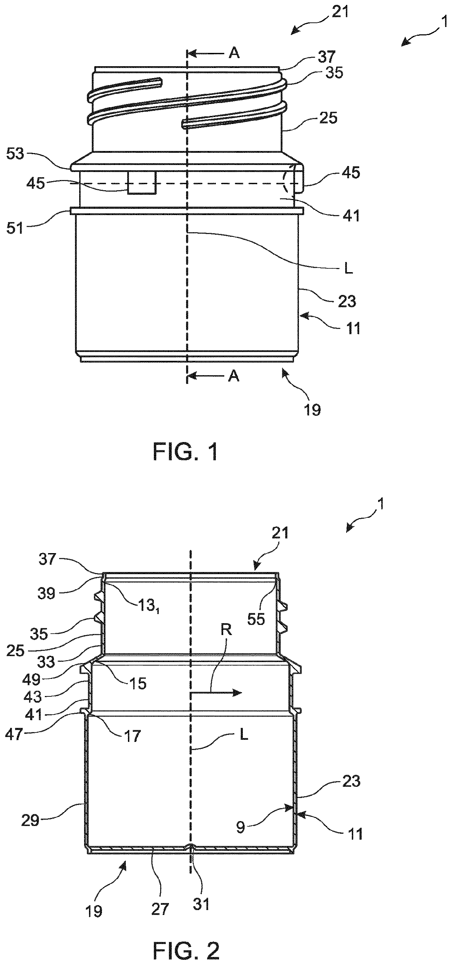

Figures 1 and 2 show a primary packaging container 1 for pharmaceutical, medical, cosmetic, dietetic and/or food products. The container 1 comprises an interieur surface 9 and an exterior surface 11. The interieur surface 9 comprises three undercut sections 13, 15 and 17.

The container 1 is rotationally shaped along a longitudinal axis L and comprises a closed bottom 19 at one end of the longitudinal axis L and an open top 21 at the opposite end of the longitudinal axis L.

The container 1 comprises a cylindrical body portion 23 extending from the closed bottom 19 towards the open top 21. The body portion 23 comprises a disc shaped bottom 27 and a jacket 29 with a hollow cylindrical shape. The interieur surface and the exterior surface of the jacket 29 of the body portion 23 is flat, i.e. free of recesses, protrusions and/or threads. The interieur surface and the exterior surface of the bottom 27 of the body portion 23 is, except of a bending 31 in its center caused by the injection nozzle, also flat, i.e. free of recesses, protrusions and/or threads. The interieur diameter of the jacket 29 of the body portion 23 is 39,39 mm. By means of a small tapering, the interieur diameter of the bottom 27 of the body portion 23 is slightly smaller, namely 38,8 mm. The interieur of the body portion 23 extends along the longitudinal axis L along 27 mm. The wall thickness of the body portion 23 is constantly 0,5 mm.

The container 1 further comprises a cylindrical neck portion 25 extending between the body portion 23 portion and the open top 21. The neck portion 25 comprises a jacket 33 with a hollow cylindrical shape. The interieur surface of the jacket 33 of the neck portion 25 is flat, i.e. free of recesses, protrusions and/or threads. The exterior surface of the jacket 33 of the neck portion 25 comprises thread means 35 for a closure. The interieur diameter of the jacket 33 of the neck portion 25 is 33 mm. The interieur of the neck portion 25 extends along the longitudinal axis L along 14,36 mm. The wall thickness of the neck portion 25 is, except of the thread means, constantly 0,5 mm.

The container 1 further comprises a cylindrical mouth portion 37 extending between the open top 21 and the neck portion 25. The mouth portion 37 comprises a jacket 39 with a hollow cylindrical shape. The interieur and the exterior surface of the jacket 39 of the mouth portion 37 is flat, i.e. free of recesses, protrusions and/or threads. The interieur diameter of the jacket 39 of the mouth portion 37 is 32,4 mm. The interieur of the mouth portion 37 extends along the longitudinal axis L along 1 mm. The wall thickness of the mouth portion 37 is constantly 0,5 mm.

The container 1 further comprises a cylindrical safety-lock portion 41 extending between the neck portion 25 and the body portion 23. The safety-lock portion 41 comprises a jacket 43 with a hollow cylindrical shape. The interieur surface of the jacket 43 of the safety-lock portion 41 is flat, i.e. free of recesses, protrusions and/or threads. The exterior surface of the jacket 33 of the safety-lock portion 41 comprises safety lock means 45 for a closure. The interieur diameter of the jacket 43 of the safety-lock portion 41 is 38 mm. The interieur of the safety-lock portion 41 extends along the longitudinal axis L along 14,36 mm. The wall thickness of the neck portion 25 is, except of the safety lock means 45, constantly 0,5 mm.

The container 1 further comprises a lower part 47 of a shoulder portion extending between the safety-lock portion 41 and the body portion 23 and an upper part 49 of a shoulder portion extending between the safety-lock portion 41 and the neck portion 25. The inside surface of the lower part 47 and of the upper part 49 of the shoulder portion has a truncated cone shape tapering towards the open top 21. Thereby, the lower part 47 of the shoulder portion realizes the undercut section 17 while the upper part 49 of the shoulder portion realizes the undercut section 15. The outside surface of the lower part 47 and the upper part 49 of the shoulder portion each comprise a ring-shaped protrusion 51, 53 protruding in radial direction R beyond the exterior surface of the body portion 23 and of the safety-lock portion 41. The ring-shaped protrusions 51, 53 provide the safety-lock portion 41 with an upper and a lower rim for guiding safety lock means of a closure being intended to interact with safety lock means 45 of the safety-lock portion 41.

The container 1 further comprises a portion 55 between the mouth portion 37 and neck portion 25 having a truncated cone shape tapering towards the open top 21, thereby, realizing the undercut section 13.

The interieur diameter, namely 39,38 mm, of the body portion 23 divided by the interieur diameter, namely 32,4 mm, of the mouth portion 37 defines an overall undercut ratio of the container 1 of 1,215. The interieur diameter, namely 39,38 mm, of the body portion 23 divided by the interieur diameter, namely 38 mm, of the safety-lock portion 41 defines a body/safety-lock ratio of 1,036. The interieur diameter, namely 38 mm, of the safety-lock portion 41 divided by the interieur diameter, namely 33 mm, of the neck portion 25 defines a safety-lock/neck ratio of 1,152. The interieur diameter, namely 33 mm, of neck portion 25 divided by the interieur diameter, namely 32,4 mm, of the mouth portion 37 defines a neck/mouth ratio of 1,019.

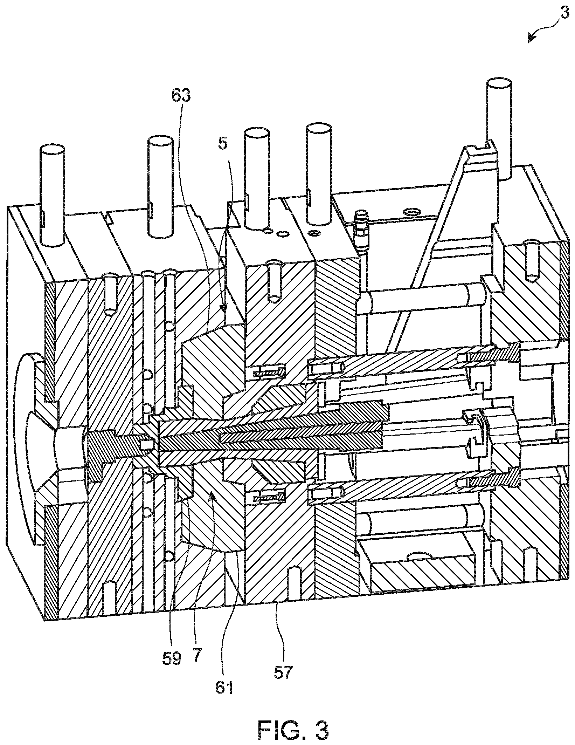

Figure 3 is a cross section view of an apparatus 3 for injection molding the container 1 shown in figure 1 and 2. The apparatus 3 is illustrated in a position, in which a molding chamber 55 is formed between the cavity member 5 and the core member 7. This position can also be referred to as injection position.

In the apparatus 3 of figure 3, in accordance with step a) of the inventive method, a cavity member 5 is provided defining an exterior surface of the container 1. In the present case, the cavity member 5 comprises a top cavity part 57, a bottom cavity part 59 and two intermediate cavity parts 61, 63. The top cavity part provides the previously described ring-shaped exterior top surface of the molding chamber 55. The bottom cavity part 59 provides the previously described exterior surface sections for the formation of the body portion 23 of the later container 1. The two intermediate cavity parts 61, 63 provide the exterior surface section for the formation of the previously described safety-lock portion 41, neck portion 25 and mouth portion 37. The top cavity part 57 and the bottom cavity part 59 are movable along the longitudinal axis L, while the intermediate cavity parts 61, 63 are movable in radial direction R.

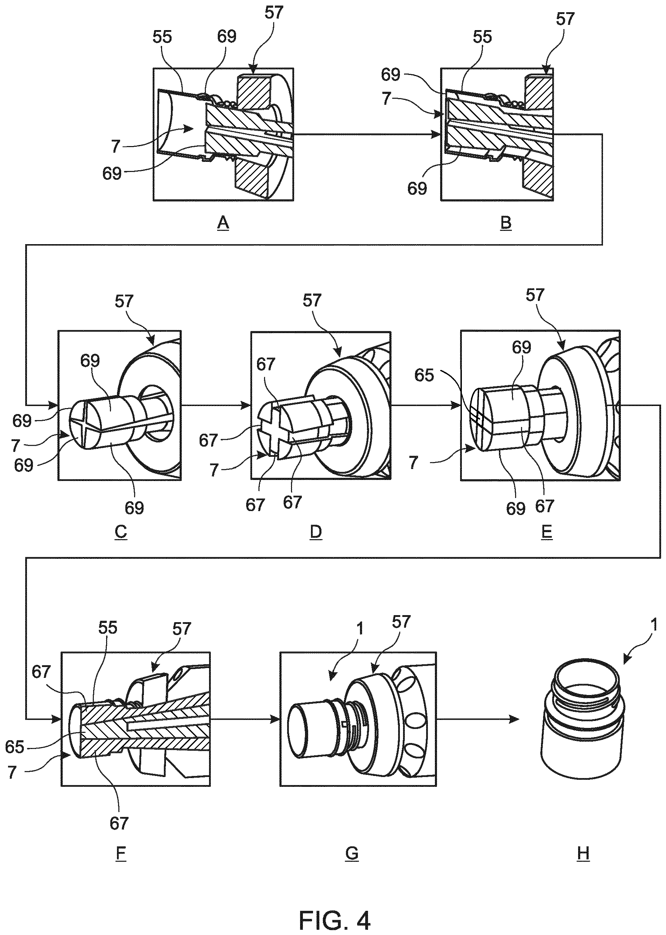

Further, in accordance with step b) of the inventive method, a core member 7 defining an interior surface 9 of the container 1 with at least one undercut section 13, 15, 17, namely the three undercut sections 13, 15, 17 (not shown in figure 3), is provided. The structure of the core member 7 and how it can be brought into the injection position c) will be described in more detail with respect to Figures 4 to 7.

For bringing the cavity member 5 in step c) into the injection position, the top cavity part 57 is moved along the longitudinal axis L towards the open top 21 of the later container 1, the bottom cavity part 59 is moved along the longitudinal axis L towards the closed bottom 19 of the later container 1, wherein the intermediate cavity parts 61, 63 are moved in radial direction R towards the longitudinal axis L of the later container 1. Preferably, the cavity member 5 is brought into the injection position before the core member 7.

Figure 4 shows illustrations A to G of different positions of the core member 7 between step c) and d) of the inventive method and the inventive container 1 produced thereby in illustration G. In illustrations A to G, except of a section of the top cavity part 57 of the cavity member 5, the cavity member 5 is faded out to better show the core member 7. However, in the illustrations A, B and F, the interieur and exterior surface of the molding chamber 55 is indicated by sectionally fading in the later container 1 which is therein referred to with reference sign 55 to make clear that it only illustrates the molding chamber 55 in the injection position. In illustration G the container 1 being produced after injection of material into the molding chamber 55 is illustrated and referred to with reference sign 1.

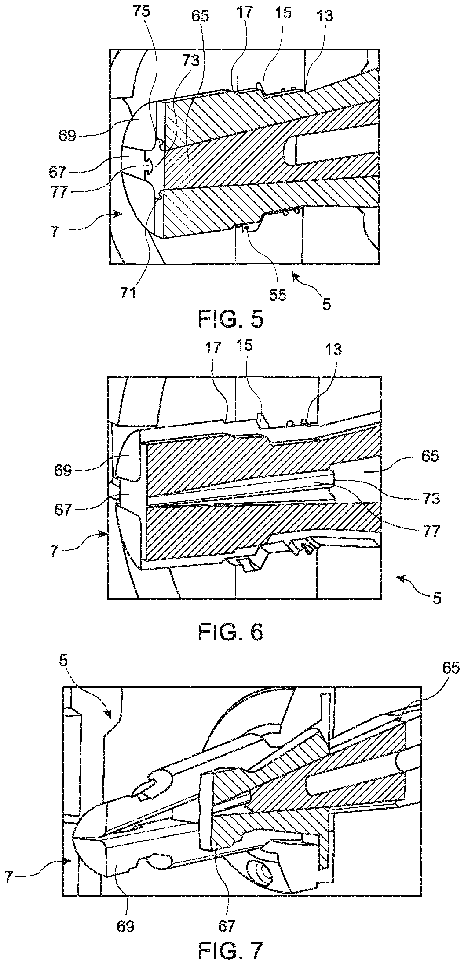

As will be described in more detail with respect to Figures 5 to 7, the core member 7 comprises a central portion 65, four rod portions 67 and four cylinder segment portions 69.

As described above, the core member 7 is preferably brought into the injection position after the cavity member 5. Illustrations A to E of Figure 4 show different positions adopted by the central portion 65, the four rod portions 67 and four cylinder segment portions 69 when bringing the core member 7 in step c) into the injection position, if the cavity member 5 is already in injection position. Illustration A shows the beginning of inserting the core member 7, wherein the four cylinder segment portions 69 (due to the cut out view, only two can be seen) are inserted through the open top 21 while being tilted in radial direction inwards to be able to pass the open top 21. Illustrations B and C show the four cylinder segment portions 69 being in their final position along the longitudinal axis L but still tilted inwards, i.e. in collapsed state. Illustration D shows a position of the core member 7, in which the four rod portions 67 have been inserted through the open top 21 into their final position along the longitudinal axis L in that they push the cylinder segment portions 69 in radial direction T outwards so that they are partially expanded. Illustration E shows a position of the core member 7, in which the central portion 65 is inserted through the open top 21 into its final position along the longitudinal axis L in that it pushes the rod portions 67 in radial direction R outwards into their final radial position in radial direction R, whereby also the cylinder segment portions 69 are pushed in radial direction R outwards into their final radial position in radial direction R, so that the core member 7 is in its completely expanded state, in which the cavity member 5 and the core member 7 form in between them the molding chamber 55, according to step c). Illustration F schematically stands for step d), namely injecting material into the molding chamber 55 for molding the container 1 and shows a cross section view of the core member 7, in particular of two rod portions 67 and the central portion 65. Illustration G schematically stands for step e), namely removing the core member 7 from the interior of the container 1 by collapsing the core member 7 to allow the core member 7 passing the undercut sections 13, 15, 17, which step is described in detail with respect to figures 5 to 7. Illustration H shows the injected molded container 1 after the cavity member 5 has been removed from the molding position and after the container 1 has been ejected from the apparatus 3, for example by a handling robot gripping the container before the last part of the cavity member 5 is removed.

Figures 5 to 7 show detailed cross section views of the core member 7 in different positions, namely in the injection position (Figure 5), at the beginning of collapsing it for passing the undercut section in step e); and in the middle of collapsing it for passing the undercut section in step e). In Figures 5 and 6, the bottom cavity part 59 of the cavity member 5 is faded out for better presentation of the core member 7. In Figure 7, the bottom cavity part 59 and the intermediate cavity parts 61 and 63 are faded out to better show the core member 7.

As can be seen in Figure 5, in the injection position, the central portion 65 forms a part of the closed bottom of the interieur surface of the molding chamber 55. The central portion 65 has a truncated cone shape tapering towards the closed bottom of the molding chamber 55.

The four rod portions 67 protrude radially from the central portion 65 and are tapered towards the open top of the molding chamber 55 with the cross section profile of the molding chamber 55, i.e. of the interieur surface of the later container 1. The rod portions 67 and the central portion 65 are movable relative to each other along the longitudinal axis L. For this purpose, a longitudinal guidance is provided between the central portion 65 and rod portions 67. The linear guidance is realized by a tongue-groove guidance. For this purpose, two of the rod portions 67 comprise a groove 71 and the central portion 65 comprises two tongues 75 engaging with the two grooves 71. Further the central portion comprises two grooves 73 and two of the rod portions 67 comprises tongues 77 engaging with the two grooves 73 of the central portion 65.

The four cylinder segment portions 69 protrude in circumferential direction between the rod portions 67 and are tapered towards the open top of the molding chamber 55 with the cross section profile of the molding chamber 55, i.e. of the interieur surface of the later container 1. The cylinder segment portions 69 and the rod portions 67 are movable relative to each other along the longitudinal axis L. For this purpose a longitudinal guidance is provided between the rod portions 67 and the cylinder segment portion 69. The linear guidance is realized in that each cylinder segment portions 69 is in circumferential direction bordered by two rod portions 67 acting as shoulders for guiding the cylinder segment portion 69 in between.

By comparing Figure 5 and 6, it can be seen that removing the core member 7 from the container 1 in step e) can comprise in s first sub-step removing the central portion 65 in opening direction through the open top 21 of the container 1 which allows the rod portions 67 to tilt in radial direction R inwards, i.e. to collapse, thereby reducing their exterior extension which allows the rod portions 67 to pass the undercut sections 13, 15 and 17.

By comparing Figure 6 and 7 it can be seen that removing the core member 7 from the container 1 in step e) can comprise in a second sub-step removing the rod portion 67 in opening direction through the open top 21 of the container 1 which allows the cylinder segment portions 69 to tilt in radial direction R inwards, i.e. to collapse, thereby reducing their exterior extension which allows the cylinder segment portion 69 to pass the undercut sections 13, 15 and 17.

In a third, not shown, sub-step, the cylinder segment portions 69 can be removed in opening direction through the open top 21 of the container 1 to complete removing the core member 7 from the container in step e).

The features disclosed in the foregoing description, the figures, and the claims may be significant, both individually and in any combination, for the realization of the invention in the various embodiments.

### REFERENCE SIGNS:

- 1: container
- 3: apparatus
- 5: cavity member
- 7: core member
- 9: interieur surface
- 11: exterior surface
- 13, 15, 17: undercut section
- 19: closed bottom
- 21: open top
- 23: body portion
- 25: neck portion
- 27: bottom
- 37: mouth portion
- 29, 33, 39, 43: jacket
- 41: safety-lock portion
- 45: safety lock means
- 47: lower part
- 49: upper part
- 51, 53: ring-shaped protrusion
- 55: molding chamber
- 57: top cavity part
- 59: bottom cavity part
- 61, 63: intermediate cavity parts
- 65: central portion
- 67: rod portion
- 69: cylinder segment portion
- 71, 73: groove
- 75, 77: tongue

- L: longitudinal axis
- R: radial direction

## Claims

1. A container (1), in particular a primary packaging container, for pharmaceutical, medical, cosmetic, dietetic and/or food products, comprising an interior surface with at least one undercut section (13, 15, 17), wherein at least a portion of the container has an average wall thickness of maximally 0,8 mm, preferably of between 0,2 mm and 0,8 mm, more preferably between 0,3 mm and 0,7 mm, most preferably between 0,4 mm and 0,6 mm.

2. A container (1), in particular a primary packaging container, for pharmaceutical, medical, cosmetic, dietetic and/or food products, in particular according to claim 1, comprising an interior surface with at least one undercut section (13, 15, 17), wherein at least a portion of the container has a wall thickness with a standard deviation of less than 0,02 mm, preferably of less than 0,015 mm, more preferably of less than 0,01 mm, even more preferably of less than 0,005 mm, most preferably of less than 0,0025 mm, for example of about 0,001 mm.

3. The container according to claim 1 or 2, wherein the at least one portion is selected from a closed bottom, a body portion extending between the closed bottom and an open top, a neck portion extending between the body portion and the open top, a safety-lock portion extending between the body portion and the neck portion and a shoulder portion extending between the body portion and the neck portion.

4. A container (1), in particular a primary packaging container, for pharmaceutical, medical, cosmetic, dietetic and/or food products, in particular according to one of the preceding claims, comprising an interior surface with at least one undercut section (13, 15, 17), wherein at least two, preferably at least three or four, portions selected from a body portion extending between a closed bottom and an open top of the container, a neck portion extending between the body portion and the open top, a safety-lock portion extending between the body portion and the neck portion and a shoulder portion extending between the body portion and the neck portion, have an average wall thickness differing from each other by less than 0,07 mm, preferably by less than 0,06 mm, more preferably by less than 0,05 mm, most preferably by less than 0,04 mm, for example by 0,03 mm.

5. The container according to any of the previous claims, wherein the container is produced by a two-step injection molding method, preferably comprising the following steps:
a) providing a cavity member (5) defining an exterior surface (11) of the container (1);
b) providing a core member (7) defining an interior surface of the container (1) with at least one undercut section (13, 15, 17);
c) bringing the cavity member (5) and the core member (7) into a position, in which a molding chamber (55) is formed between the cavity member (5) and the core member;
d) injecting material into the molding chamber (55) to mold the container (1); and
e) removing the core member (7) from the interior of the container (1).

6. A container (1), in particular a primary packaging container, for pharmaceutical, medical, cosmetic, dietetic and/or food products, in particular according to one of the preceding claims, being injection molded by a method comprising the following steps:
a) providing a cavity member (5) defining an exterior surface (11) of the container (1);
b) providing a core member (7) defining an interior surface of the container (1) with at least one undercut section (13, 15, 17);
c) bringing the cavity member (5) and the core member (7) into a position, in which a molding chamber (55) is formed between the cavity member (5) and the core member;
d) injecting material into the molding chamber (55) to mold the container (1); and
e) removing the core member (7) from the interior of the container (1), whereby the core member collapses for passing the undercut region.

7. The container (1) according to one of the preceding claims, wherein the interior of the container (1) is at least partially rotationally shaped along a longitudinal axis and comprises a closed bottom (19) at one end of the longitudinal axis and an open top (21) at the opposite end of the longitudinal axis preferably wherein the undercut section (13, 15, 17) is located between the smallest diameter of the interior and the largest diameter of the interior between the smallest diameter and the bottom, preferably wherein the largest diameter divided by the smallest diameter defines an undercut ratio of at least 1.05, preferably at least 1.10, more preferably at least 1.15, most preferably of at least 1.20.

8. The container (1) according to claim 7, comprising a body portion (23) extending from the closed bottom (19) towards the open top (21) and a neck portion (25) extending between the body portion and the open top (21), wherein the inside of the body portion (23) and the neck portion (25) is preferably cylindrically shaped, preferably wherein the diameter of the neck portion (25) divided by the diameter of the body portion (23) defines a body/neck ratio of at least 1.04, preferably of at least 1.09, more preferably of at least 1.13, most preferably of at least 1.18, preferably further comprising a mouth portion (37) extending between the open top (21) and the neck portion (25), wherein the inside of the mouth portion (37) is preferably cylindrically shaped, preferably wherein the diameter of the body portion (23) divided by the diameter of the mouth portion (37) defines a body/mouth ratio of at least 1.05, preferably of at least 1.10, more preferably of at least 1.15, most preferably of at least 1.20, and/or wherein the diameter of the neck portion (25) divided by the diameter of the mouth portion (37) defines a neck/mouth ratio of more than 1, preferably of at least 1.01, more preferably of at least 1.015.

9. The container (1) according to claim 8, further comprising a safety-lock portion (41) extending between the neck portion (25) and the body portion (23), wherein the inside of the safety-lock portion (41) is preferably cylindrically shaped, preferably wherein the diameter of the safety-lock portion (41) divided by the diameter of the neck portion (25) defines a safety-lock/neck ratio of more than 1, preferably of more than 1.04, more preferably of more than 1.08, most preferably of more than 1.12, and/or wherein the diameter of the body portion divided by the diameter of the safety-lock portion (41) defines a body/safety-lock ratio of more than 1, preferably of at least 1.02, more preferably of at least 1.03.

10. The container (1) according to one of the claims 8 to 9, further comprising a shoulder portion extending between the neck portion (25) and the body portion (23), wherein the inside of the shoulder portion has preferably a truncated cone shape tapering towards the open top, preferably wherein the shoulder portion is separated into two parts by the safety-lock portion according to claim 9, in particular in that a lower part of the shoulder portion facing the closed bottom (19) connects the body portion (23) with the safety-lock portion (41) while an upper part (49) of the shoulder portion facing the open top (21) connects the safety-lock portion with the neck portion.

11. The container (1) according to one of the claims 8 to 10, wherein the at least one undercut section is realized by a portion between the shoulder portion and the mouth portion (37) according to claim 8 and/or by the shoulder portion according to claim 10, preferably wherein the container (1) comprises at least two undercut sections being preferably realized by the portion between the neck portion (25) and the mouth portion (37) and at least a part of the shoulder portion or by an upper and lower part of the shoulder portion, more preferably wherein the container (1) comprises at least three undercut sections being realized by a portion between the neck portion (25) and the mouth portion (37), the upper part (49) of the shoulder portion and the lower part of the shoulder portion.

12. The container (1) according to one of the preceding claims, wherein the exterior of the container (1) comprises thread means, in particular at the exterior of the neck portion (25) according to claim 7, for a threaded closure, and/or safety-lock means, in particular at the exterior of the safety-lock portion (41) according to claim 9, for preventing children from removing a closure from the container (1).

13. The container (1) according to one of the preceding claims, wherein the container (1) comprises a polymer, preferably a Polyolefin, more preferably a Polyolefin selected from Polypropylene, Polyethylene, Cyclo-Olefin Polymer and/or Cyclo-Olefin Co-Polymer, most preferably Polypropylene, wherein the container preferably consists of the polymer.

14. A method for injection of molding a container (1), in particular a primary packaging container, preferably according to one of the preceding claims, for pharmaceutical, medical, cosmetic, dietetic and/or food products, wherein the method comprises the following steps:
a) providing a cavity member (5) defining an exterior surface (11) of the container (1);
b) providing a core member (7) defining an interior surface of the container (1) with at least one undercut section (13, 15, 17);
c) bringing the cavity member (5) and the core member (7) into a position, in which a molding chamber (55) is formed between the cavity member (5) and the core member (7);
d) injecting material into the molding chamber (55) to mold the container (1); and
e) removing the core member (7) from the interior of the container (1) by collapsing the core member to allow the core member (7) to pass the undercut section (13, 15, 17).

15. An apparatus (3) for injection molding of a container (1), in particular a primary packaging container (1), preferably according to one of the claims 1 to 14, for pharmaceutical, medical, cosmetic, dietetic and/or food products, wherein the apparatus (3) comprises:
a) a cavity member (5) defining an exterior surface (11) of the container (1);
b) a core member (7) defining an interior surface of the container (1) with at least one undercut section (13, 15, 17);
c) a mechanism for bringing the cavity member (5) and the core member (7) into a position, in which a molding chamber (55) is formed between the cavity member (5) and the core member (7); and
d) an injection member for injecting material into the molding chamber (55) to mold the container (1);
wherein the mechanism is adapted to remove the core member (7) from the interior of the container (1) by collapsing the core member (7) to allow the core member (7) to pass the undercut section (13, 15, 17).

## Amended claims

### Amended claims in accordance with Rule 137(2) EPC.

1. A container (1), in particular a primary packaging container, for pharmaceutical, medical, cosmetic, dietetic and/or food products, comprising an interior surface with at least one undercut section (13, 15, 17), wherein at least a portion of the container has an average wall thickness of maximally 0,8 mm, preferably of between 0,2 mm and 0,8 mm, more preferably between 0,3 mm and 0,7 mm, most preferably between 0,4 mm and 0,6 mm,
wherein the container is produced by a two-step injection molding method, preferably comprising the following steps:
a) providing a cavity member (5) defining an exterior surface (11) of the container (1);
b) providing a core member (7) defining the interior surface of the container (1) with the at least one undercut section (13, 15, 17);
c) bringing the cavity member (5) and the core member (7) into a position, in which a molding chamber (55) is formed between the cavity member (5) and the core member;
d) injecting material into the molding chamber (55) to mold the container (1); and
e) removing the core member (7) from the interior of the container (1),
and wherein the interior of the container (1) is at least partially rotationally shaped along a longitudinal axis and comprises a closed bottom (19) at one end of the longitudinal axis and an open top (21) at the opposite end of the longitudinal axis wherein the undercut section (13, 15, 17) is located between the smallest diameter of the interior and the largest diameter of the interior between the smallest diameter and the bottom, wherein the largest diameter divided by the smallest diameter defines an undercut ratio of at least 1.15, preferably at least 1.20.

2. A container (1), in particular a primary packaging container, for pharmaceutical, medical, cosmetic, dietetic and/or food products, in particular according to claim 1, comprising an interior surface with at least one undercut section (13, 15, 17), wherein at least a portion of the container has a wall thickness with a standard deviation of less than 0,02 mm, preferably of less than 0,015 mm, more preferably of less than 0,01 mm, even more preferably of less than 0,005 mm, most preferably of less than 0,0025 mm, for example of about 0,001 mm,
wherein the container is produced by a two-step injection molding method, preferably comprising the following steps:
a) providing a cavity member (5) defining an exterior surface (11) of the container (1);
b) providing a core member (7) defining the interior surface of the container (1) with the at least one undercut section (13, 15, 17);
c) bringing the cavity member (5) and the core member (7) into a position, in which a molding chamber (55) is formed between the cavity member (5) and the core member;
d) injecting material into the molding chamber (55) to mold the container (1); and
e) removing the core member (7) from the interior of the container (1),
and wherein the interior of the container (1) is at least partially rotationally shaped along a longitudinal axis and comprises a closed bottom (19) at one end of the longitudinal axis and an open top (21) at the opposite end of the longitudinal axis wherein the undercut section (13, 15, 17) is located between the smallest diameter of the interior and the largest diameter of the interior between the smallest diameter and the bottom, wherein the largest diameter divided by the smallest diameter defines an undercut ratio of at least 1.15, preferably at least 1.20.

3. The container according to claim 1 or 2, wherein the at least one portion is selected from a closed bottom, a body portion extending between the closed bottom and an open top, a neck portion extending between the body portion and the open top, a safety-lock portion extending between the body portion and the neck portion and a shoulder portion extending between the body portion and the neck portion.

4. A container (1), in particular a primary packaging container, for pharmaceutical, medical, cosmetic, dietetic and/or food products, in particular according to one of the preceding claims, comprising an interior surface with at least one undercut section (13, 15, 17), wherein at least two, preferably at least three or four, portions selected from a body portion extending between a closed bottom and an open top of the container, a neck portion extending between the body portion and the open top, a safety-lock portion extending between the body portion and the neck portion and a shoulder portion extending between the body portion and the neck portion, have an average wall thickness differing from each other by less than 0,07 mm, preferably by less than 0,06 mm, more preferably by less than 0,05 mm, most preferably by less than 0,04 mm, for example by 0,03 mm,
wherein the container is produced by a two-step injection molding method, preferably comprising the following steps:
a) providing a cavity member (5) defining an exterior surface (11) of the container (1);
b) providing a core member (7) defining the interior surface of the container (1) with the at least one undercut section (13, 15, 17);
c) bringing the cavity member (5) and the core member (7) into a position, in which a molding chamber (55) is formed between the cavity member (5) and the core member;
d) injecting material into the molding chamber (55) to mold the container (1); and
e) removing the core member (7) from the interior of the container (1),
and wherein the interior of the container (1) is at least partially rotationally shaped along a longitudinal axis and comprises a closed bottom (19) at one end of the longitudinal axis and an open top (21) at the opposite end of the longitudinal axis wherein the undercut section (13, 15, 17) is located between the smallest diameter of the interior and the largest diameter of the interior between the smallest diameter and the bottom, wherein the largest diameter divided by the smallest diameter defines an undercut ratio of at least 1.15, preferably at least 1.20.

5. A container (1), in particular a primary packaging container, for pharmaceutical, medical, cosmetic, dietetic and/or food products, in particular according to one of the preceding claims, being injection molded by a method comprising the following steps:
a) providing a cavity member (5) defining an exterior surface (11) of the container (1);
b) providing a core member (7) defining an interior surface of the container (1) with at least one undercut section (13, 15, 17);
c) bringing the cavity member (5) and the core member (7) into a position, in which a molding chamber (55) is formed between the cavity member (5) and the core member;
d) injecting material into the molding chamber (55) to mold the container (1); and
e) removing the core member (7) from the interior of the container (1), whereby the core member collapses for passing the undercut region,
wherein the interior of the container (1) is at least partially rotationally shaped along a longitudinal axis and comprises a closed bottom (19) at one end of the longitudinal axis and an open top (21) at the opposite end of the longitudinal axis wherein the undercut section (13, 15, 17) is located between the smallest diameter of the interior and the largest diameter of the interior between the smallest diameter and the bottom, wherein the largest diameter divided by the smallest diameter defines an undercut ratio of at least 1.15, preferably at least 1.20.

6. The container (1) according to any one of claims 1, 2, 4, 5, comprising a body portion (23) extending from the closed bottom (19) towards the open top (21) and a neck portion (25) extending between the body portion and the open top (21), wherein the inside of the body portion (23) and the neck portion (25) is preferably cylindrically shaped, preferably wherein the diameter of the neck portion (25) divided by the diameter of the body portion (23) defines a body/neck ratio of at least 1.04, preferably of at least 1.09, more preferably of at least 1.13, most preferably of at least 1.18, preferably further comprising a mouth portion (37) extending between the open top (21) and the neck portion (25), wherein the inside of the mouth portion (37) is preferably cylindrically shaped, preferably wherein the diameter of the body portion (23) divided by the diameter of the mouth portion (37) defines a body/mouth ratio of at least 1.05, preferably of at least 1.10, more preferably of at least 1.15, most preferably of at least 1.20, and/or wherein the diameter of the neck portion (25) divided by the diameter of the mouth portion (37) defines a neck/mouth ratio of more than 1, preferably of at least 1.01, more preferably of at least 1.015.

7. The container (1) according to claim 6, further comprising a safety-lock portion (41) extending between the neck portion (25) and the body portion (23), wherein the inside of the safety-lock portion (41) is preferably cylindrically shaped, preferably wherein the diameter of the safety-lock portion (41) divided by the diameter of the neck portion (25) defines a safety-lock/neck ratio of more than 1, preferably of more than 1.04, more preferably of more than 1.08, most preferably of more than 1.12, and/or wherein the diameter of the body portion divided by the diameter of the safety-lock portion (41) defines a body/safety-lock ratio of more than 1, preferably of at least 1.02, more preferably of at least 1.03.

8. The container (1) according to one of the claims 6 to 7, further comprising a shoulder portion extending between the neck portion (25) and the body portion (23), wherein the inside of the shoulder portion has preferably a truncated cone shape tapering towards the open top, preferably wherein the shoulder portion is separated into two parts by the safety-lock portion according to claim 7, in particular in that a lower part of the shoulder portion facing the closed bottom (19) connects the body portion (23) with the safety-lock portion (41) while an upper part (49) of the shoulder portion facing the open top (21) connects the safety-lock portion with the neck portion.

9. The container (1) according to one of the claims 6 to 8, wherein the at least one undercut section is realized by a portion between the shoulder portion and the mouth portion (37) according to claim 6 and/or by the shoulder portion according to claim 8, preferably wherein the container (1) comprises at least two undercut sections being preferably realized by the portion between the neck portion (25) and the mouth portion (37) and at least a part of the shoulder portion or by an upper and lower part of the shoulder portion, more preferably wherein the container (1) comprises at least three undercut sections being realized by a portion between the neck portion (25) and the mouth portion (37), the upper part (49) of the shoulder portion and the lower part of the shoulder portion.

10. The container (1) according to one of the preceding claims, wherein the exterior of the container (1) comprises thread means, in particular at the exterior of the neck portion (25) according to any one of claims 1, 2, 4, 5, for a threaded closure, and/or safety-lock means, in particular at the exterior of the safety-lock portion (41) according to claim 7, for preventing children from removing a closure from the container (1).

11. The container (1) according to one of the preceding claims, wherein the container (1) comprises a polymer, preferably a Polyolefin, more preferably a Polyolefin selected from Polypropylene, Polyethylene, Cyclo-Olefin Polymer and/or Cyclo-Olefin CoPolymer, most preferably Polypropylene, wherein the container preferably consists of the polymer.

12. A method for injection of molding a container (1), in particular a primary packaging container, preferably according to one of the preceding claims, for pharmaceutical, medical, cosmetic, dietetic and/or food products, wherein the method comprises the following steps:
a) providing a cavity member (5) defining an exterior surface (11) of the container (1);
b) providing a core member (7) defining an interior surface of the container (1) with at least one undercut section (13, 15, 17);
c) bringing the cavity member (5) and the core member (7) into a position, in which a molding chamber (55) is formed between the cavity member (5) and the core member (7);
d) injecting material into the molding chamber (55) to mold the container (1); and
e) removing the core member (7) from the interior of the container (1) by collapsing the core member to allow the core member (7) to pass the undercut section (13, 15, 17),
wherein the interior of the container (1) is at least partially rotationally shaped along a longitudinal axis and comprises a closed bottom (19) at one end of the longitudinal axis and an open top (21) at the opposite end of the longitudinal axis wherein the undercut section (13, 15, 17) is located between the smallest diameter of the interior and the largest diameter of the interior between the smallest diameter and the bottom, wherein the largest diameter divided by the smallest diameter defines an undercut ratio of at least 1.15, preferably at least 1.20.

13. An apparatus (3) for injection molding of a container (1), in particular a primary packaging container (1), preferably according to one of the claims 1 to 12, for pharmaceutical, medical, cosmetic, dietetic and/or food products, wherein the apparatus (3) comprises:
a) a cavity member (5) defining an exterior surface (11) of the container (1);
b) a core member (7) defining an interior surface of the container (1) with at least one undercut section (13, 15, 17);
c) a mechanism for bringing the cavity member (5) and the core member (7) into a position, in which a molding chamber (55) is formed between the cavity member (5) and the core member (7); and
d) an injection member for injecting material into the molding chamber (55) to mold the container (1);
wherein the mechanism is adapted to remove the core member (7) from the interior of the container (1) by collapsing the core member (7) to allow the core member (7) to pass the undercut section (13, 15, 17),
and wherein the interior of the container (1) is at least partially rotationally shaped along a longitudinal axis and comprises a closed bottom (19) at one end of the longitudinal axis and an open top (21) at the opposite end of the longitudinal axis preferably the undercut section (13, 15, 17) is located between the smallest diameter of the interior and the largest diameter of the interior between the smallest diameter and the bottom, wherein the largest diameter divided by the smallest diameter defines an undercut ratio of at least 1.15, preferably at least 1.20.
